# EUROPEAN PATENT APPLICATION

(11) **EP 2 380 562 A1**
(43) Date of publication of application: **26.10.2011**
(21) Application number: 09832823.0
(22) Date of filing: 16.12.2009
(51) Int. Cl.: A61K 9/22, A61K 9/28, A61K 31/4422, A61P 9/10

(54) **A CONTROLLED-RELEASED OSMOTIC PUMP TABLET WITH LUBRICATING LAYER AND THE PREPARATION THEREOF**

(30) Priority: 16.12.2008 CN 200810239714
(71) Applicant: Beijing Kexin Bicheng Biomedical Technology Development Co., Ltd., Beijing 100190 (CN)
(72) Inventor: JIANG, Haisong, Beijing 100190 (CN); WANG, Jingang, Beijing 100190 (CN)
(74) Representative: Crowhurst, Charlotte Waveney
(86) International application number: PCT/CN2009/001473
(87) International publication number: WO 2010/069141

(57) **Abstract**

The invention relates to the field of pharmaceutical preparation, in particular to a bilayer osmotic pump controlled-release tablet preparation and a preparation method thereof. Said bilayer osmotic pump controlled-release tablet comprises a conventional tablet core, release control layer(s) and drug release hole(s), wherein one or more lubricating layer structure(s) is/are disposed between the tablet core and the release control layer(s). The lubricating layer(s) is/are present in an amount of from 0.5% to 6% based on the weight of the bilayer tablet core. The lubricating layer(s) rapidly hydrates once in contact with water to form the favorable lubricating liquor between the tablet core and the release control layer(s), hence reducing the shear force between the drug-containing layer(s) and the release control layer(s), whereby the drug-containing layer(s) is/are pushed out completely by the propellant layer(s) to reach a drug release percent of nearly 100%. This solves the problem of the incomplete drug release due to the dead angle generated by the drug-containing layer of a conventional bilayer osmotic pump controlled-release tablet during the release procedure. This technique ensures the superior bioavailability of the bilayer osmotic pump controlled-release tablet as prepared.

## Description

### Technical Field

The invention relates to the field of pharmaceutical preparation, in particular to a bilayer osmotic pump controlled-release tablet preparation and a preparation method thereof.

### Background Art

An osmotic pump controlled-release tablet is a typical controlled release preparation of zero-order release. In 1974, Theeuwes invented a single-chamber osmotic pump, which was in a simple form of ordinary coated tablet with one side being drilled. Thus, osmotic pump became a dosage form that could be used in clinic, and therefore a series of single-chamber osmotic pump controlled-release tablet preparation were developed. From 1980's, the modification and development about osmotic pump never stopped. In order to prepare insoluble drug into osmotic pump, or to prepare drug with good water solubility but cannot produce osmotic pressure by itself into osmotic pump, a single-chamber bilayer osmotic pump was developed, which solved the technical problem of preparing an insoluble drug into an osmotic pump controlled-release tablet. However, the problem of incomplete drug release occurs frequently during the application of the bilayer osmotic pump controlled-release tablet technique. It is prescribed in the Chinese Pharmacopoeia in regard to the fundamental requirements on the sustained-release preparations and the controlled-release preparations that the release platform of the sustained-release preparations and the controlled-release preparations should reach a release amount of more than 90%, but currently a lot of bilayer osmotic pumps controlled-release preparation do not meet this requirement. In the meantime, the incomplete release of a drug seriously affects the therapeutic effects and bioavailability of the drug.

Reasons for the incomplete release of a drug are as follows. Polyethylene oxide (PEO) is an indispensable material for the preparation of the bilayer osmotic pump controlled-release tablet, and it swells once in contact with water and is further hydrated to form a non-Newtonian fluid, pushed by a propellant layer and discharged together with the drug from the drug release hole of the osmotic pump controlled-release tablet, such that the drug is released at a constant speed. Since the drug layer containing polyethylene oxide forms a non-Newtonian fluid after being hydrated, it can be determined from the fundamental properties of a non-Newtonian fluid that a high shear force exists between the non-Newtonian fluids formed by the side wall of the release control layer and the drug-containing layer in the osmotic pump controlled-release tablet, and the drug layer adjacent to the translucent film side wall has a migration rate of almost 0 when pushed by the propellant layer, hence forming a dead angle of drug release, which results in that this part of drug cannot be completely pushed out by the propellant layer, the drug cannot be completely released, and the bioavailability of the drug is relatively low, which seriously affects the therapeutic effects of the drug. In order to remove the disadvantages of the bilayer osmotic pump controlled-release tablet, a bilayer osmotic pump controlled-release tablet having a lubricating layer structure is developed.

### Contents of Invention

A first object of the present invention is to prepare a bilayer osmotic pump controlled-release tablet capable of completely releasing a drug, which has an improved release effect compared with a conventional bilayer osmotic pump controlled-release tablet; another object of the present invention is to provide a process for preparing said bilayer osmotic pump controlled-release tablet.

In order to achieve the above objects, the present invention adopts the following technical solution:
A bilayer osmotic pump controlled-release tablet, which comprises a bilayer tablet core containing a drug, one or more release control layer(s) around the tablet core and one or more drug release hole(s) on the release control layer(s), wherein one or more lubricating layer structure is/are disposed between the tablet core and the release control layer(s).

The bilayer tablet core containing a drug, release control layer(s) and drug release hole(s) according to the present invention may be those conventionally used, such as a drug-containing bilayer tablet core consisting of one or more drug-containing layer(s) and one or more propellant layer(s), and one or more drug release hole(s) disposed on the release control layer(s) at the drug-containing layer side.

The drug-containing layer(s) and propellant layer(s) according to the present invention may be those conventionally used. The common materials used in the field of osmotic pump controlled-release tablet for preparing drug-containing layers and propellant layers may be used. As generally known, the materials for the propellant layer include those which swell once in contact with water, hence pushing the drug layer out of the drug release hole(s) of the osmotic pump controlled-release tablet. In one embodiment, the materials for the propellant layer include polyethylene oxide (PEO). In another embodiment, besides polyethylene oxide, the materials for the propellant layer further include cellulose, preferably hydroxypropylmethyl cellulose. In one embodiment, the materials for the drug-containing layer include polyvidone. In another embodiment, besides polyvidone, the materials for the drug-containing layer further include cellulose, preferably hydroxypropylmethyl cellulose.

In one embodiment, the high-substituted hydroxypropyl cellulose has a viscosity of from 2.4 to 120 mPa·S, preferably from 12 to 80 mPa·S.

In one embodiment, the low-viscosity hydroxypropylmethyl cellulose has a viscosity of from 5 to 100 mPa·S, preferably from 5 to 50 mPa·S.

In one embodiment, the drug-containing layer comprises a drug and polyvidone. In another embodiment, the drug-containing layer comprises a drug, polyvidone and cellulose, preferably hydroxypropylmethyl cellulose.

In one embodiment, the propellant layer comprises polyethylene oxide. In another embodiment, the propellant layer comprises polyethylene oxide and cellulose, preferably hydroxypropylmethyl cellulose.

In one preferred technical solution of the present invention, the material for the lubricating layer is selected from a high-substituted hydroxypropyl cellulose or a low-viscosity hydroxypropylmethyl cellulose, which is present in an amount of from 0.5% to 6% based on the weight of the bilayer tablet core. The main material for the release control layer is cellulose acetate, which is present in an amount of from 18% to 24% based on the weight of the bilayer tablet core. The drug release hole has a diameter between 0.4 mm and 1.2 mm.

In the present invention, a lubricating layer(s) is/are added between the tablet core and the release control layer(s). After a patient takes the osmotic pump controlled-release tablet, water in the alimentary tract penetrates the release control layer(s) and comes into contact with the lubricating layer(s), which rapidly hydrate(s) to become a liquid gel having superior flowability and lubricating effect, hence forming one or more lubricating layer having a lubricating effect between the tablet core and the release control layer(s) and reducing the shear force between the drug-containing layer and release control layer of the bilayer tablet, whereby the drug in the drug-containing layer(s) is/are pushed out successfully and completely by the propellant layer(s) to reach a drug release percent of nearly 100% within 24 hours.

A process for preparing the bilayer osmotic pump controlled-release tablet of the present invention comprises the steps of preparing a drug-containing bilayer tablet core from a drug and auxiliaries, first coating the tablet core with one or more lubricating layer(s), then coating the lubricating layer with one or more release control layer(s), and then drilling one or more hole(s) at the drug-containing layer side to form the bilayer osmotic pump controlled-release tablet. An ordinary film coat(s) may be optionally applied on the outer surface of the release control layer(s) in regard to practical production needs. The lubricating layer(s), release control layer(s) and film coat(s) can all be prepared by a high-efficiency coating machine.

The above-mentioned process for preparing a bilayer osmotic pump controlled-release tablet comprises the following specific steps:
(1) Preparing the bilayer tablet core containing drug:
   The material for the drug-containing layer and the material for the propellant layer are pressed by a bilayer tablet machine to produce a bilayer tablet core.
(2) Preparing the coating liquor for the lubricating layer(s):
   A suitable amount of a material for the lubricating layer is added into water or ethanol or a solution of water-ethanol at any ratio, impregnated, and stirred to be dissolved. The material for the lubricating layer(s) is selected from a high-substituted hydroxypropyl cellulose or a low-viscosity hydroxypropylmethyl cellulose. Since hydroxypropyl cellulose and hydroxypropylmethyl cellulose are soluble both in water and in ethanol, they are dissolvable in a solution of water-ethanol at any ratio. The concentration of the coating liquor for the lubricating layer(s) is controlled between 2% to 20%. A solvent with a higher water content has a higher coating temperature during coating, while a solution with a higher ethanol content has a lower coating temperature but a high cost with safety issues. Thus, drugs unstable at high temperatures cannot be coated with a coating liquor having a higher water content, and can be coated with a coating liquor having a higher ethanol content, even a coating liquor containing ethanol as the solvent; as to drugs insensitive to temperature, a solvent with a higher water content, even a solvent system which is completely water, can be used.
(3) Coating the lubricating layer(s):
   The bilayer tablet is placed in a high-efficiency coating machine and coated with the coating liquor for the lubricating layer(s), the load (i.e., the percent of the weight of the lubricating layer(s) relatively to the weight of the bilayer tablet core) of which is controlled between 0.5% and 6%. The air intake temperature of the coating machine is controlled between 40 °C and 65 °C.
(4) Preparing the coating liquor for the release control layer(s):
   Cellulose acetate is added into acetone, impregnated and stirred to be dissolved to prepare a solution comprising from 1% to 6% (w/w) of cellulose acetate, in which a pore-forming agent and a plasticizer are added and mixed uniformly. The pore-forming agent is selected from the group consisting of polyethylene glycol, polyvidone (PVP), lactose, sucrose, mannitol and sorbitol. The plasticizer is selected from the group consisting of dibutyl sebacate, phthalate and triethyl citrate.
(5) Coating the release control layer(s):
   The bilayer tablet coated with the lubricating layer(s) is put into the high-efficiency coating machine and coated with the coating liquor for the release control layer(s), till the load (i.e., the percent of the weight of the release control layer(s) relatively to the weight of the bilayer tablet core) of the release control layer(s) is from 18% to 24%. The air intake temperature of the coating machine is controlled between 15 °C and 35 °C.
(6) Aging the release control layer(s):
   The bilayer tablet coated with the release control layer(s) is put into a drying apparatus to be dried. The drying temperature is controlled between 35 °C and 60 °C, and the drying does not stop until the residual acetone is less than 0.05%.
(7) Drilling one or more hole(s):
   The aged controlled-release tablet is drilled at the drug-containing layer side by laser drilling or mechanical drilling. The hole diameter is controlled between 0.4 mm and 1.2 mm, and one or two holes may be drilled.
(8) Coating a film coat(s):
   The film coat(s) is/are optionally applied onto the outer surface of the drilled bilayer osmotic pump controlled-release tablet by a conventional coating technique.

The bilayer osmotic pump controlled-release tablet according to the present invention further comprises one or more lubricating layer(s), as compared with conventional bilayer osmotic pump controlled-release tablet. Said lubricating layer(s) consists of materials that easily hydrate and forms a gel film having a lubricating effect after being hydrated, so has a superior lubricating effect. When the propellant layer absorbs water and swells, pushing the drug layer upward, the whole drug layer migrates thanks to the lubricating effect of the lubricating layer(s) without any dead angle formed, such that the drug layer is finally completely pushed out, and the drug is completely released. The lubricating layer(s) consists of a high-substituted hydroxypropyl cellulose or a low-viscosity hydroxypropylmethyl cellulose. A conventional coating method is useful for the preparation of a lubricating layer(s) by coating the whole bilayer tablet core. The steps are simple and the operation is easy, but the drug release effect is significantly improved without any effects on the coating procedure of the release control layer(s). In other words, a big problem is solved by the simplest method.

In one preferred embodiment, the bilayer osmotic pump controlled-release tablet according to the present invention is a bilayer osmotic pump controlled-release tablet of nifedipine, wherein the drug contained in the bilayer tablet core is nifedipine.

### Drawings of Description

Fig. 1 is a structural representation of the bilayer osmotic pump controlled-release tablet according to the present invention, which has a lubricating layer structure, wherein reference sign 1 represents a drug layer, reference sign 2 represents a propellant layer, reference sign 3 represents a lubricating layer, reference sign 4 represents a release control layer, and reference sign 5 represents a drug release hole.
Fig. 2 is a graph showing the release effect of Example 1.
Fig. 3 shows the effect of the amount of the lubricating layer on release.
Fig. 4 shows the effect of the load of the release control membrane on release.
Fig. 5 shows the effect of the solvent in the lubricating layer on release.

### Examples

The present invention is illustrated by the following Examples. It should be understood that these Examples are merely used for illustration of the present invention and do not limit the scope of the present invention.

### Example 1 An osmotic pump controlled-release tablet of nifedipine having a lubricating layer structure

An osmotic pump controlled-release tablet of nifedipine having a lubricating layer structure was prepared by the following steps:
1. Pulverization of a nifedipine raw material:
A nifedipine raw material was pulverized to obtain a pulverized raw drug having a particle size of less than 10 µm.

2. Preparation of a material comprising a nifedipine solid micropowder:
Nifedipine: polyvidone K30: water of 6: 12: 80 (w/w) were mixed, and formed a stable, uniform aqueous dispersion suspension with a high-speed dispersion machine. The suspension was spray dried to prepare a solution comprising a nifedipine solid micropowder, which was dried at a temperature between 40 °C and 50 °C for posterior use.

3. Granulation of materials for a drug-containing layer:
Formulation of the drug-containing layer is following.

| | |
|---|---|
| Nifedipine | 30.0g |
| Polyvidone K30 | 60.0g |
| Polyethylene oxide | 40.0g |
| Hydroxypropylmethyl cellulose | 20.0g |
| Magnesium stearate | 1.0g |
| Silica gel micropowder | 0.75g |
| | 1000 tablets |

1) A solution of a nifedipine-polyvidone solid micropowder, polyethylene oxide, and hydroxypropylmethyl cellulose were taken from the materials in the formulation of the drug-containing layer and dry granulated.
2) Mixing: the resultant particles were mixed with the silica gel micropowder and magnesium stearate till they were uniformly mixed for posterior use.
4. Mixing of materials for a propellant layer:
Formulation of the propellant layer is following.

| | |
|---|---|
| Polyethylene oxide | 48.0g |
| Hydroxypropylmethyl cellulose | 24.0g |
| Sodium chloride | 5.0g |
| Iron oxide red | 2.0g |
| Magnesium stearate | 0.7g |
| | 1000 tablets |

Polyethylene oxide, hydroxypropylmethyl cellulose, sodium chloride, iron oxide red and magnesium stearate were mixed together till they were uniformly mixed for posterior use.
5. Press of a bilayer tablet:
The drug-containing layer particles and propellant layer materials that had been uniformly mixed were processed into a bilayer tablet by 8.5 mm shallow concave compressing to obtain a tablet core for posterior use.

6. Preparation of a lubricating layer coating liquor:
Formulation of the lubricating layer is following.

| | |
|---|---|
| High-substituted hydroxypropyl cellulose | 5 % |
| Ethanol | 95 % |

5g Hydroxypropyl cellulose was added into 95g ethanol and stirred to be dissolved for posterior use.
7. Coating of a lubricating layer:
The pressed bilayer tablet was coated with a lubricating layer by a high-efficiency coating machine. The temperature at the tablet bed was controlled to 45 °C ± 2 °C during the coating procedure, and the load of the lubricating layer was 1 %.

8. Preparation of a release control membrane coating liquor:
Formulation of the release control layer is following.

| | |
|---|---|
| Cellulose acetate | 3.20 % |
| Polyethylene glycol 1500 | 0.48 % |
| Dibutyl sebacate | 0.32 % |
| Acetone | 96.0% |

Cellulose acetate, polyethylene glycol 1500 and dibutyl sebacate were added into acetone in turn and stirred to be dispersed. After the solids completely swelled, keep stirring till they were dissolved for posterior use.
9. Coating of a release control layer:
The tablet that had been coated with a shielding coat was coated with a release control membrane by the high-efficiency coating machine. The coating procedure did not need to heat the material bed, and the load of the coat was controlled to 21 % (w/w) relative to the bilayer tablet core.

10. Aging of the release control layer:
The tablet coated with the release control layer was put into oven and aged for 16 hours at 40 °C to 45 °C.

11. Laser drilling:
The aged tablet was taken out and drilled by a laser driller at the center of the drug-containing layer to obtain a drug release hole having a diameter of 700 µm.

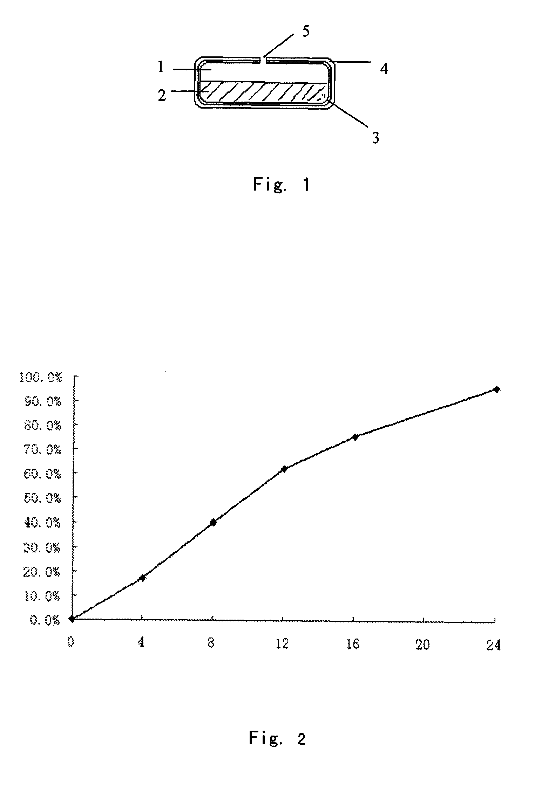

The resultant osmotic pump controlled-release tablet of nifedipine having a lubricating layer structure had a structure as shown in Fig. 1, in which a drug-containing layer 1 and a propellant layer 2 form a bilayer tablet core, a lubricating layer 3 and a release control layer 4 are present in turn on the bilayer tablet core, and a drug release hole 5 is present at the drug-containing layer side of the controlled-release tablet.

### Release effect of Example 1:

The release effect was measured by the first method described in the Chinese Pharmacopoeia for measuring a release percent, with the apparatus in the second method for measuring dissolution test. The release medium is 600 ml phosphate-citric acid buffer solution (pH=6.8) containing 1% of sodium lauryl sulfate. The rotate speed is 100 turns/minute. The HPLC method was used for measuring the release percent under the following chromatographic conditions: the fixed phase was C18, and the mobile phase was acetonitrile/methanol/H₂O (20:30:50). The release result as measured was a release percent of higher than 90% within 24 hours, which is in conformity with the general provision on the sustained-release preparations and the controlled-release preparations in the Chinese Pharmacopoeia. The release graph is as shown in Fig. 2.

| Time (h) | 0 | 4 | 8 | 12 | 16 | 24 |
|---|---|---|---|---|---|---|
| Release | | | | | | |
| percent | 0.0% | 17.3% | 40.1% | 62.3% | 75.8% | 96.0% |

### Example 2 Effect of the amount of the lubricating layer on release

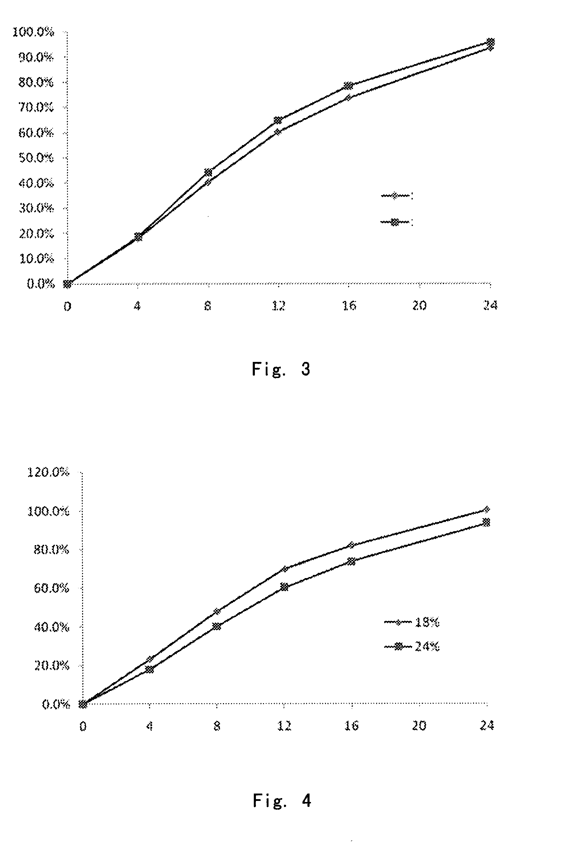

An osmotic pump controlled-release tablet of nifedipine was prepared according to Example 1 from the same amounts of the same materials by the same steps. The difference was that the loads of the lubricating layer were respectively 0.5% and 6%. The effect of the amount of the lubricating layer on release was evaluated. The release result is in conformity with the general provision on the sustained-release preparations and the controlled-release preparations in the Chinese Pharmacopoeia, which was a release percent of higher than 90% within 24 hours. The release graph as measured is as shown in Fig. 3.

| Time (h) | 0 | 4 | 8 | 12 | 16 | 24 |
|---|---|---|---|---|---|---|
| Load 0.5% | 0.0% | 17.8% | 40.1% | 60.2% | 73.6% | 93.3% |
| Load 6% | 0.0% | 18.3% | 44.1% | 64.7% | 78.3% | 95.7% |

Conclusion: The lubricating layer could effectively play its lubricating role when the load of the lubricating layer was between 0.5% and 6%.

### Example 3: Effect of the load of the release control membrane on release

An osmotic pump controlled-release tablet of nifedipine was prepared according to Example 1 from the same amounts of the same materials by the same steps. The difference was that the loads of the release control membrane coat were respectively 18% and 24%. The release result is in conformity with the general provision on the sustained-release preparations and the controlled-release preparations in the Chinese Pharmacopoeia, which was a release percent of higher than 90% within 24 hours. The release graph is as shown in Fig. 4.

| Time (h) | 0 | 4 | 8 | 12 | 16 | 24 |
|---|---|---|---|---|---|---|
| 18% | 0.0% | 23.3% | 47.8% | 69.7% | 81.8% | 100.3% |
| 24% | 0.0% | 17.8% | 40.1% | 60.2% | 73.6% | 93.3% |

Conclusion: The samples had superior release percents when the load of the release control membrane coat was between 18% and 24%, and the release percent within 24 hours could reach more than 90% when the load was 24%, which is in conformity with the provision in the Chinese Pharmacopoeia.

### Example 4 Effect of the solvent of the lubricating layer on release

A bilayer tablet was prepared according to the process described in Example 1, and lubricating layer coating liquors having two different solvents were formulated according to the following formulations.

### Formulation of a lubricating layer 1:

| | |
|---|---|
| Hydroxypropylmethyl cellulose | 5% |
| Water | 95% |

Method of formulation: 5g Hydroxypropylmethyl cellulose was added in 95g water and stirred to be dissolved for posterior use.

### Formulation of a lubricating layer 2:

| | |
|---|---|
| Hydroxypropylmethyl cellulose | 5g |
| ethanol | 75g |

Method of formulation: 5g Hydroxypropylmethyl cellulose was added in 75g ethanol and stirred to be dissolved for posterior use.

Coating of a lubricating layer: The pressed bilayer tablets were put into a high-efficiency coating machine and coated with the lubricating layer 1 and the lubricating layer 2, respectively. The temperature at the tablet bed was controlled to 45 ± 2 °C during the coating procedures, and the load of the lubricating layers was about 2%.

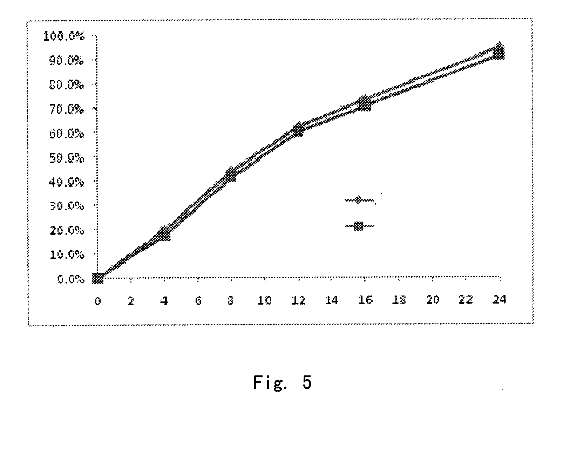

Posterior steps according to the preparation process described in Example 1 were carried out. The loads of the release control layers were respectively 21.3% and 22.1%. The release percent was measured according to the method described in Example 1. The release results of the osmotic pump controlled-release tablets of nifedipine prepared from two different lubricating layer solvents are both in conformity with the general provision on the sustained-release preparations and the controlled-release preparations in the Chinese Pharmacopoeia, which were release percents of higher than 90% within 24 hours. The measured results are as show below. The release graph is as shown in Fig. 5.

| Time (h) | 4 | 8 | 12 | 16 | 24 |
|---|---|---|---|---|---|
| Water | 20.5% | 44.0% | 66.4% | 72.1% | 94.3% |
| Ethanol | 18.7% | 43.4% | 64.1% | 70.6% | 91.5% |

Conclusion: Osmotic pump controlled-release tablets with stable release could be prepared from both water and ethanol which were used as the solvents of the lubricating layer coating liquor, and the release percents of the two tablets were not significantly different.

## Claims

1. A bilayer osmotic pump controlled-release tablet, which comprises a bilayer tablet core containing a drug, one or more release control layer(s) around the tablet core and one or mone drug release hole(s) on the release control layer(s), **characterized in that** one or more lubricating layer structure(s) is/are disposed between the tablet core and the release control layer.

2. The bilayer osmotic pump controlled-release tablet according to claim 1, **characterized in that** the material for the lubricating layer(s) is/are selected from a high-substituted hydroxypropyl cellulose or a low-viscosity hydroxypropylmethyl cellulose.

3. The bilayer osmotic pump controlled-release tablet according to claim 2, **characterized in that** the high-substituted hydroxypropyl cellulose has a viscosity of from 2.4 to 120 mPa·S, preferably from 12 to 80 mPa·S.

4. The bilayer osmotic pump controlled-release tablet according to claim 2, **characterized in that** the low-viscosity hydroxypropylmethyl cellulose has a viscosity of from 5 to 100 mPa·S, preferably from 5 to 50 mPa·S.

5. The bilayer osmotic pump controlled-release tablet according to any of claims 1 to 4, **characterized in that** the drug-containing bilayer tablet core consists of one or more drug-containing layer(s) and one or more propellant layer(s).

6. The bilayer osmotic pump controlled-release tablet according to any of claims 1 to 5, **characterized in that** the lubricating layer(s) is/are present in an amount of from 0.5% to 6% based on the weight of the bilayer tablet core.

7. The bilayer osmotic pump controlled-release tablet according to any of claims 1 to 6, **characterized in that** the release control layer(s) is/are present in an amount of from 18% to 24% based on the weight of the bilayer tablet core.

8. The bilayer osmotic pump controlled-release tablet according to any of claims 1 to 7, **characterized in that** the release control layer(s) is/are made from cellulose acetate.

9. The bilayer osmotic pump controlled-release tablet according to any of claims 1 to 8, **characterized in that** the drug release hole(s) thereof has/have a diameter between 0.4mm and 1.2mm.

10. The bilayer osmotic pump controlled-release tablet according to any of claims 1 to 13, which is a bilayer osmotic pump controlled-release tablet of nifedipine, wherein the drug contained in the bilayer tablet core is nifedipine.

11. A process for preparing a bilayer osmotic pump controlled-release tablet according to any of claims 1 to 10, comprising the steps of preparing a drug-containing, bilayer tablet core from a drug and auxiliaries, first coating the tablet core with one or more lubricating layer(s), then coating the lubricating layer with one or more release control layer(s), and then drilling hole(s) at the drug-containing layer side of the release control layer(s) to form the bilayer osmotic pump controlled-release tablet.

12. The process according to claim 11 for preparing a bilayer osmotic pump controlled-release tablet, **characterized in that** said process comprises the following steps:
(1) preparing a drug-containing bilayer tablet core, wherein the material for the drug-containing layer and the material for the propellant layer are pressed by a bilayer tablet machine to produce a bilayer tablet core;
(2) preparing the coating liquor for the lubricating layer(s), wherein the suitable amount of the material for the lubricating layer(s) is added into water or ethanol or a solution of water-ethanol at any ratio, impregnated, and stirred to be dissolved, and the material for the lubricating layer(s) is selected from a high-substituted hydroxypropyl cellulose or a low-viscosity hydroxypropylmethyl cellulose;
(3) coating the lubricating layer(s), wherein the bilayer tablet(s) is/are placed in a high-efficiency coating machine and coated with the lubricating layer coating liquor;
(4) preparing the coating liquor for the release control layer(s), wherein cellulose acetate is added into acetone, impregnated and stirred to be dissolved to prepare a solution comprising from 1% to 6% (w/w) of cellulose acetate, in which a pore-forming agent and a plasticizer are added and mixed uniformly;
(5) coating the release control layer(s), wherein the bilayer tablet coated with the lubricating layer(s) is put into the high-efficiency coating machine and coated with the release control layer coating liquor;
(6) aging the release control layer(s), wherein the bilayer tablet coated with the release control layer(s) is put into a drying apparatus to be dried, the drying temperature is controlled between 35 °C and 60 °C, and the drying does not stop until the residual acetone is less than 0.05%.
(7) drilling one or more hole(s), wherein the aged controlled release tablet is drilled at the drug-containing layer side of the release control layer(s).

13. The process according to claim 12 for preparing a bilayer osmotic pump controlled-release tablet, **characterized in that** said process comprises step (8) coating a film coat(s), wherein a film coat(s) is/are applied onto the outer surface of the drilled bilayer osmotic pump controlled-release tablet by a conventional coating technique.

14. The process according to any of claims 11 to 13 for preparing a bilayer osmotic pump controlled-release tablet, wherein the bilayer osmotic pump controlled-release tablet is a bilayer osmotic pump controlled-release tablet of nifedipine, wherein the drug contained in the bilayer tablet core is nifedipine.
